Europäisches Patentamt

**(19)** European Patent Office

Office européen des brevets

**(11)** **EP 0 589 934 B1**

**(12)** **FASCICULE DE BREVET EUROPEEN**

**(45)** Date de publication et mention
de la délivrance du brevet:
**08.10.1997 Bulletin 1997/41**

**(21)** Numéro de dépôt: 92911627.5

**(22)** Date de dépôt: 04.06.1992

**(51)** Int. Cl.[6]: **A61K 7/48**, A61K 35/78

**(86)** Numéro de dépôt international:
**PCT/FR92/00494**

**(87)** Numéro de publication internationale:
**WO 92/21322 (10.12.1992 Gazette 1992/31)**

**(54)** **COMPOSITION COSMETIQUE OU PHARMACEUTIQUE, NOTAMMENT DERMATOLOGIQUE, CONTENANT UN EXTRAIT DE BRUNELLE**

PRUNELLA-EXTRAKT ENTHALTENDE KOSMETISCHE ODER PHARMAZEUTISCHE, INSBESONDERE DERMATOLOGISCHE, ZUSAMMENSETZUNG

COSMETIC OR PHARMACEUTICAL, PARTICULARLY DERMATOLOGICAL, COMPOSITION CONTAINING A PRUNELLA EXTRACT

**(84)** Etats contractants désignés:
**BE CH DE ES FR GB IT LI**

**(30)** Priorité: **04.06.1991 FR 9106757**

**(43)** Date de publication de la demande:
**06.04.1994 Bulletin 1994/14**

**(73)** Titulaire: **LVMH RECHERCHE**
**92752 Nanterre (FR)**

**(72)** Inventeurs:
• **BONTE, Frédéric**
**F-92400 Courbevoie (FR)**
• **MEYBECK, Alain Les Poissons**
**F-92400 Courbevoie (FR)**
• **DUMAS, Marc**
**F-92700 Colombes (FR)**

**(74)** Mandataire: **Portal, Gérard et al**
**Cabinet Beau de Loménie**
**158, rue de l'Université**
**75340 Paris Cédex 07 (FR)**

**(56)** Documents cités:
• **Patent Abstracts of Japan, vol. 13, no. 315 (C-619), 18 juillet 1989, & JP,A, 1102027 (NITTO DENKO CORP.) 19 avril 1989, voir le résumé**
• **Patent Abstracts of Japan, vol. 11, no. 300 (C-449), 29 septembre 1987, & JP,A, 62093217 (SHISEIDO) 28 avril 1987, voir le résumé**
• **WPIL, Serveur de Bases de Donnees, AN=91-033705 [05], Derwent Publications Ltd, Londres, GB, & JP-A-2304009 (POLA CHEM. IND. K.K.) 17 décembre 1990, voir le résumé**
• **Parfums, Cosmétiques, Arômes, no. 69, juin-juillet 1986, (Paris, FR), O. SCHMIDIGER: "Extraits végétaux: produits cosmétiques et phytopharmaceutiques", pages 51-54, voir page 52, tableau 1**

Printed by Rank Xerox (UK) Business Services
2.14.16/3.4

**Description**

La présente invention concerne essentiellement une composition cosmétique ou pharmaceutique, notamment dermatologique, contenant un extrait de Brunelle.

Plus précisément, la présente invention concerne l'utilisation d'un extrait de Brunelle ou d'au moins une saponine de Brunelle pour la préparation d'une composition cosmétique ou pharmaceutique, notamment dermatologique, en particulier à activité anti-inflammatoire, anti-vieillissement, favorisant ou régulant le renouvellement cellulaire cutanée, de prévention et de traitement de la chute des cheveux ou des alopécies, ainsi qu'une composition cosmétique en comportant application.

Les plantes du genre Brunelle, également désignées sous le nom générique de Prunella en particulier Prunella Vulgaris L. ou "herbe au charpentier" encore dénommée Brunelle commune ou petite Consoude sont des Labiées communes dans les pays de la zone tempérée, notamment en France. De manière classique, elle était utilisée fraîche, avec son suc notamment sous forme de gargarisme pour traiter les angines, la toux.

Elle a également été utilisée en tisane contre les hémorragies, les maladies des voies digestives. Plus récemment, il a été décrit par Lee et Lin dans Mutation Research, 204 (1988), 229-234, une activité antimutagène d'extraits de Prunella Vulgaris L.

Le document Patent Abstract of Japan, vol. 13, N°315 (C-619), décrit un produit pharmaceutique ayant des propriétés anti-allergiques obtenu à partir de Prunella Vulgaris.

Le document Patent Abstract of Japan, vol. 11, N°300 (C-449), divulgue une composition cosmétique comprenant à titre d'ingrédient actif une ou plusieurs saponines extraites de manière conventionnelle de Prunella Vulgaris, à une concentration comprise entre 0,001 et 10 % en poids parmi de nombreux autres ingrédients actifs, utilisée comme tonique pour les cheveux.

On sait que les effets du vieillissement sur la peau se caractérisent en particulier par un ralentissement de la différenciation cellulaire au niveau cutané, d'où un ralentissement du renouvellement des cellules, de leur activité, de leur morphologie, et, en ce qui concerne les kératinocytes, un ralentissement de leur transformation en cornéocytes, ce qui entraîne un aspect de la peau plus terne, desséché et plus ridé. Les effets du vieillissement au niveau des follicules pileux sont également négatifs. Ils entraînent pour les cellules des follicules, comme pour celles de l'épiderme, une dimension de leur activité, d'où un ralentissement de la croissance des cheveux, et, à terme, une dégénérescence du follicule et la perte définitive du cheveu.

La présente invention a donc pour but principal de résoudre le nouveau problème technique consistant en la fourniture d'une nouvelle formulation d'une composition cosmétique ou pharmaceutique, notamment dermatologique, présentant une bonne efficacité sur le renouvellement de l'épiderme, la repousse des cheveux, la prévention ou le ralentissement de leur chute, ainsi que dans la lutte contre les effets du vieillissement sur l'état de la peau, de la chevelure, et des ongles.

La présente invention a encore pour but principal de résoudre le nouveau problème technique consistant en la fourniture d'une nouvelle formulation d'une composition cosmétique ou pharmaceutique et/ou dermatologique, à activité anti-inflammatoire, en particulier vis-à-vis de l'érythème provoqué par les rayonnements ultraviolets.

La présente invention a pour but principal de résoudre les nouveaux problèmes techniques énoncés ci-dessus d'une manière particulièrement simple, ne nécessitant pas obligatoirement l'isolement d'une substance active particulière ou sa synthèse, satisfaisante et utilisable à l'échelle industrielle.

Ainsi, selon un premier aspect, la présente invention concerne l'utilisation d'un extrait de Brunelle ou d'au moins une saponine de Brunelle pour la préparation d'une composition pharmaceutique, notamment dermatologique, présentant une activité anti-radicalaire, une activité anti-inflammatoire, notamment dans les inflammations provoquées par le rayonnement ultraviolet, régulant le renouvellement et la différenciation des kératinocytes, permettant d'améliorer ou de maintenir un bon état fonctionnel de la peau ou des phanères, de prévenir ou de traiter des peaux sèches, notamment des peaux ichtyosiques, ou de traiter le psoriasis.

Egalement, la présente invention concerne l'utilisation d'un extrait de Brunelle ou d'au moins une saponine de Brunelle, comme agent cosmétique destiné à améliorer ou maintenir un bon état fonctionnel de la peau ou des phanères, à lutter contre les effets du vieillissement de l'épiderme ou des phanères, à prévenir, à traiter les peaux sèches, notamment les peaux ichtyosiques, ou à lutter contre les inflammations, notamment l'érythème provoqué par les rayonnements ultraviolets.

Egalement, la présente invention couvre l'utilisation d'un extrait de Brunelle ou d'au moins une saponine de Brunelle pour favoriser ou réguler la différenciation et le renouvellement des cellules de l'épiderme en culture.

Selon une variante de réalisation particulièrement préférée, la Brunelle précitée est la Prunella Vulgaris L. ou Brunelle commune.

Selon une variante particulière, l'extrait de Brunelle précité ou la saponinie de Brunelle précitée est obtenu par extraction à partir de la plante entière, ou de préférence, à partir des parties aériennes de Brunelle. De préférence encore, les parties de la plante utilisées sont séchées préalablement au traitement d'extraction.

En particulier, l'extrait de Brunelle précité peut être obtenu selon le procédé décrit ci-après à titre indicatif, mais nullement limitatif. On effectue une extraction primaire de la matière sèche, constituée de préférence de parties aériennes de Brunelle, au moyen d'un solvant choisi parmi le groupe constitué par : l'eau, les alcools comportant de préférence de 1 à 4 atomes de carbone, les solvants chlorés comportant de préférence 1 à 2 atomes de carbone, les esters organiques comportant de préférence de 3 à 6 atomes de carbone ; ou d'un solvant mixte à base d'un mélange quelconque des solvants précités.

Avantageusement, le solvant d'extraction primaire est l'eau, le méthanol, l'éthanol, un mélange méthanol-eau ou un mélange éthanol-eau, le chloroforme, le dichlorométhane. De préférence, il s'agit de l'eau ou du méthanol.

Le rapport de la matière végétale à l'agent d'extraction n'est pas critique et généralement sera compris entre 1 : 5 et 1 : 20 parties en poids.

L'extraction primaire précitée s'effectue à des températures comprises entre la température ambiante et le point d'ébullition du solvant utilisé pour l'extraction.

De préférence, l'extraction primaire est effectuée au reflux sous pression atmosphérique pendant une durée de 2 à 4 h. En outre, elle est avantageusement précédée d'une macération à froid pendant 2 à 4 h dans le solvant d'extraction.

En fin d'extraction, la phase du solvant contenant l'extrait est filtrée puis concentrée et/ou évaporée à sec sous pression réduite. On obtient ainsi un premier extrait de Brunelle selon l'invention.

Suivant une variante particulière, l'utilisation selon l'invention est relative à un mélange de saponines de Brunelle. On obtient en particulier un mélange de saponines de Brunelle selon l'invention à partir du premier extrait de Brunelle précité concentré ou sec, en opérant comme indiqué ci-après. Le premier extrait précité est introduit puis agité dans un solvant apolaire de préférence miscible avec le solvant d'extraction primaire, tel qu'un éther ou une cétone de faible poids moléculaire, en particulier l'éther éthylique ou isopropylique, l'acétone ou la méthyl-éthyl-cétone. La quantité de solvant apolaire est, en poids, généralement de 5 à 100 parties pour une partie d'extrait primaire. L'insoluble et/ou le précipité formé contient principalement un mélange de saponines selon l'invention.

Avantageusement, le mélange de saponines obtenu précédemment est purifié par un procédé quelconque à la portée de l'homme de l'art.

En particulier l'insoluble et/ou le précipité précité est remis en solution dans 20 fois environ son poids d'eau. La solution aqueuse est ensuite extraite 3 à 4 fois par un alcool peu soluble dans l'eau, tel que le butanol saturé en eau, par exemple en proportion 1/1 en volume à chaque opération d'extraction. Les phases alcooliques sont réunies et évaporées sous pression réduite. Le résidu est dissous dans 10 fois environ son poids d'eau, la solution est ensuite dialysée contre de l'eau pure pendant 4 à 5 jours. Le contenu de la cellule de dialyse est lyophilisé. Eventuellement pour améliorer encore la purification du mélange de saponines obtenu, le lyophilisat est dissous dans du méthanol, puis jeté dans de l'éther éthylique. On recueille le précipité formé.

Selon un second aspect, la présente invention concerne aussi une composition cosmétique ou pharmaceutique, notamment dermatologique, de préférence topique, caractérisée en ce qu'elle comprend à titre d'ingrédient actif, une quantité cosmétiquement ou pharmaceutiquement efficace d'un extrait de Brunelle ou d'au moins une saponine de Brunelle, ainsi que des phases lamellaires lipidiques hydratées ou des liposomes incorporant ou non l'extrait de Brunelle ou une saponine de Brunelle, éventuellement dans un excipient cosmétiquement ou pharmaceutiquement acceptable.

Selon un autre mode de réalisation, l'invention concerne une composition cosmétique ou pharmaceutique, notamment dermatologique, régulant le renouvellement et la différenciation des kératinocytes, pour lutter contre le vieillissement cutané, pour améliorer ou maintenir un bon état fonctionnel de la peau, en particulier le rôle de barrière de protection, caractérisée en ce qu'elle comprend à titre d'ingrédient actif une quantité cosmétiquement ou pharmaceutiquement efficace d'un extrait de Brunelle ou d'au moins une saponine de Brunelle, ainsi que des phases lamellaires lipidiques hydratées ou des liposomes incorporant ou non l'extrait de Brunelle ou une saponine de Brunelle, éventuellement dans un excipient cosmétiquement ou pharmaceutiquement acceptable.

Selon encore un autre mode de réalisation de l'invention, celle-ci concerne une composition cosmétique ou pharmaceutique, notamment dermatologique, à activité anti-radicalaire, caractérisée en ce qu'elle comprend à titre d'ingrédient actif une quantité cosmétiquement ou pharmaceutiquement efficace d'un extrait de Brunelle ou d'au moins une saponine de Brunelle, ainsi que des phases lamellaires lipidiques hydratées ou des liposomes incorporant ou non l'extrait de Brunelle ou une saponine de Brunelle, éventuellement dans un excipient cosmétiquement ou pharmaceutiquement acceptable.

Selon un autre mode de réalisation, l'invention concerne encore une composition cosmétique ou pharmaceutique, notamment dermatologique, pour la prévention ou le traitement des peaux sèches, notamment des peaux ichtyosiques, caractérisée en ce qu'elle comprend à titre d'ingrédient actif, une quantité cosmétiquement ou pharmaceutiquement efficace d'un extrait de Brunelle ou d'au moins une saponine de Brunelle, ainsi que des phases lamellaires lipidiques hydratées ou des liposomes incorporant ou non l'extrait de Brunelle ou une saponine de Brunelle, éventuellement dans un excipient cosmétiquement ou pharmaceutiquement acceptable.

La présente invention concerne encore une composition pharmaceutique, notamment dermatologique, pour le traitement du psoriasis, caractérisée en ce qu'elle comprend à titre d'ingrédient actif une quantité pharmaceutiquement efficace d'un extrait de Brunelle ou d'au moins une saponine de Brunelle, ainsi que des phases lamellaires lipidiques hydratées ou des liposomes incorporant ou non l'extrait de Brunelle ou une saponine de Brunelle, éventuellement dans un excipient pharmaceutiquement acceptable.

Selon une autre variante avantageuse de l'invention, la composition cosmétique ou pharmaceutique, notamment dermatologique selon l'invention, comprend en outre au moins une autre substance active, en une concentration efficace, choisie parmi les xanthines, les vitamines, en particulier les vitamines A, B et E ou leurs dérivés comme par exemple les esters de vitamine A, la tyrosine ou ses dérivés comme par exemple le tyrosinate de glucose, la malyl tyrosine, la quinine ou ses dérivés, les rubéfiants tels que le nicotinate de méthyle, un surnageant de culture de fibroblastes de papilles, tel que décrit dans le document EP-A-272 920, des hydrolysats de kératine, des oligo-éléments tels que zinc, sélénium, cuivre, un alcaloïde bisbenzylisoquinoléique tel que l'oxyacanthine ou la cépharanthine, des inhibiteurs de 5-$\alpha$-réductase tels que : progestérone, acétate de cyprotérone, Minoxidil, acide azélaïque et ses dérivés, 1,4-diméthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

Les compositions cosmétiques ou pharmaceutiques, notamment dermatologiques, conformément à la présente invention, en particulier se présentant sous forme de crèmes, de laits, de gels ou de lotions, peuvent être appliquées par voie topique pour réguler le renouvellement et la différenciation des kératinocytes, pour lutter contre les effets du vieillissement sur l'état de la peau, pour améliorer ou maintenir un bon état fonctionnel de la peau ou des phanères, pour prévenir ou traiter les peaux sèches, pour traiter le psoriasis.

Sous cet aspect, la présente invention fournit également une méthode de traitement cosmétique pour lutter contre les effets du vieillissement sur l'épiderme ou les phanères, pour lutter contre les inflammations, notamment l'érythème provoqué par les rayonnements ultraviolets, pour améliorer ou maintenir un bon état fonctionnel de la peau ou des phanères, pour prévenir ou traiter les peaux sèches, ou pour traiter les désordres causés par l'action des radicaux libres, caractérisée en ce qu'elle comprend l'application topique, en une quantité efficace pour réaliser ledit effet recherché, d'au moins un extrait de Brunelle ou d'au moins une saponine de Brunelle, éventuellement incorporé dans un excipient cosmétiquement acceptable.

Dans tous les aspects précédents, la concentration en extrait de Brunelle ou en saponine de Brunelle est avantageusement comprise entre 0,001 % et 5 % en poids de la composition totale.

Les proportions indiquées dans la description et dans les revendications s'entendent en poids sec lorsqu'il s'agit d'extraits végétaux.

Dans tous les aspects précédents, selon une variante de réalisation particulièrement avantageuse de l'invention, la composition cosmétique ou pharmaceutique, notamment dermatologique, selon l'invention peut également contenir des phases lamellaires lipidiques hydratées ou des liposomes, incorporant ou non l'extrait de Brunelle précédemment défini.

Le terme "Lipidique" dans l'expression "phase lamellaire lipidique" couvre toutes les substances comprenant une chaîne carbonée dite grasse, comportant généralement plus de 5 atomes de carbone, cette substance étant habituellement dénommée "lipide".

Selon l'invention, on utilise à titre de lipides, pour former la phase lamellaire lipidique, ou les vésicules type liposomes, des lipides amphiphiles, c'est-à-dire constitués de molécules possédant un groupe hydrophile indifféremment ionique ou non ionique et un groupe lipophile, ces lipides amphiphiles étant susceptibles de former une phase lamellaire lipidique ou des vésicules type liposomes, en présence d'une phase aqueuse, selon la quantité d'eau dans le mélange.

En particulier, parmi ces lipides, on peut citer : les phospholipides, les phosphoaminolipides, les glycolipides, les alcools gras polyoxyéthylénés, les esters de polyol éventuellement polyoxyéthylénés. De telles substances sont par exemple constituées par une lécithine d'oeuf ou de soja, une phosphatidylsérine, une sphyngomyéline, un cérébroside, un glycosyl céramide ou un stéarate de polyglycérol oxyéthyléné.

De préférence, selon l'invention, on utilise un mélange lipidique constitué d'au moins un lipide amphiphile et d'au moins un lipide hydrophobe tel que stérol, comme le cholestérol ou le bêta-sitostérol. La quantité, exprimée en poids, de composés hydrophobes ne doit généralement pas être supérieure à la quantité de lipides amphiphiles, et de préférence ne doit pas être supérieure à 0,5 fois cette quantité.

Suivant une variante préférée, l'extrait de Brunelle ou la saponine précité est introduit dans la phase aqueuse de la phase lamellaire lipidique hydratée ou des liposomes à une concentration comprise entre 0,001 % et 5 % en poids du poids total de ladite phase aqueuse.

Suivant encore une autre variante préférée, l'extrait de Brunelle ou la saponine précité est incorporé au moins en partie dans la phase lipidique de la phase lamellaire lipidique hydratée ou des liposomes à une concentration comprise de préférence entre 0,01 % et 30 % en poids de ladite phase lipidique, et de préférence encore comprise entre 0,01 % et 10 % en poids de cette phase.

Il est précisé que l'expression "incorporé au moins en partie" couvre le cas où la substance est totalement incorporée et celui où une certaine quantité seulement de cette substance est incorporée.

L'incorporation dans des phases lamellaires lipidiques hydratées ou dans des liposomes, de la saponine précitée ou de la sapogénine correspondante ou de l'extrait de Brunelle selon l'invention, peut être réalisée selon les techniques de préparation connues, décrites par exemple dans le document EP-B1-0087 993 = US-A-4 508 703, et éventuellement en combinaison avec le document EP-B1-0 101 559 = US-A-4 621 023.

Selon une variante de réalisation du procédé de fabrication précité, celui-ci comprend en premier lieu l'ajout à un extrait de Brunelle ou à la saponine de Brunelle, de phases lamellaires lipidiques hydratées ou de liposomes, avec ou sans incorporation dans lesdites phases ou dans lesdits liposomes dudit extrait de Brunelle ou de ladite saponine de Brunelle ; puis l'ajout de L'ensemble ainsi obtenu dans un excipient, véhicule ou support pharmaceutiquement ou cosmétiquement acceptable.

D'autres buts, caractéristiques et avantages de l'invention apparaîtront clairement à la lecture de la description explicative qui va suivre, faite en référence à plusieurs exemples donnés uniquement à titre d'illustration.

Dans les exemples, les pourcentages sont exprimés en poids, sauf indication contraire. Dans le cas des extraits, les quantités de ceux-ci sont exprimées en poids sec.

Exemple 1

Préparation d'un extrait méthanolique de Brunelle

Environ 500 g de feuilles de Prunella Vulgaris L. ou Brunelle commune, séchées à l'air et pulvérisées sont mis à macérer pendant environ 2 h dans 1,2 l de méthanol. Le mélange est porté au reflux pendant 3 h. Après refroidissement, la solution est filtrée sur verre fritté n° 3. Les marcs sont extraits de nouveau de la même façon par 2 fois 1 l de méthanol. Les trois filtrats sont réunis et concentrés à l'évaporateur rotatif.

On obtient ainsi un extrait méthanolique de Brunelle selon l'invention. Si on le souhaite, on peut obtenir un extrait sec en plaçant l'extrait précédemment obtenu à l'étuve, entre 40°C et 60°C environ, jusqu'à obtention d'un poids constant.

Exemple 2

Préparation d'une suspension de liposomes gélifiés contenant un extrait de Brunelle

On dissout dans 100 ml d'un mélange chloroforme-méthanol 9:1, 9 g de lécithine de soja, 1 g de β-sitostérol et 1 g d'extrait sec de Brunelle préparé selon l'exemple 1.

La solution est évaporée sous pression réduite à 60°C dans un ballon rotatif, selon la méthode de Bangham, bien connue de l'homme de l'art du domaine des liposomes, jusqu'à obtention du dépôt d'un film lipidique sur la surface interne du ballon rotatif.

On introduit ensuite dans le ballon 89 g d'eau distillée. On réalise une agitation jusqu'à dispersion complète du film lipidique dans l'eau, à température ambiante, ce qui nécessite en général en pratique au moins 2 à 4 h d'agitation, puis on homogénéise aux ultrasons à 4°C pendant 10 min sous puissance de 150 W.

La taille moyenne des liposomes obtenus est d'environ 100 nm.

De préférence, on gélifie cette suspension de liposomes en la mélangeant à 100 g d'un gel de Carbopol 940$^R$ à 1,25% préparé séparément de façon classique. On obtient ainsi environ 200 g d'une suspension gélifiée de liposomes incorporant une majeure partie de l'extrait de Brunelle précité. La concentration en cet extrait est de 0,5 % environ par rapport au poids total de la suspension gélifiée.

On peut observer qu'en réalisant diverses dilutions, on peut obtenir des compositions de proportions variables en extraits de Brunelle, ce qui constitue un mode de formulation particulièrement aisé.

Exemple 3

Essai d'activité sur le renouvellement de l'épiderme

On réalise l'ensemencement de 50 000 kératinocytes humains transformés, dans des boîtes de Petri de diamètre 35 mm dans 2 ml de milieu EMEM de Gibco, auquel on a ajouté 1 % en volume de SVF (SVF = sérum de veau foetal), sans antibiotique.

On procède à une incubation pendant 24 h à la température de 37°C.

Après ces 24 h d'incubation, on renouvelle les milieux par un milieu identique mais contenant respectivement différentes concentrations des produits à tester comme défini ci-après. Les essais sont réalisés en triple et des boîtes témoins sont prévues qui ne contiennent que le solvant du produit testé.

On réalise une incubation pendant 5 j à la température de 37°C, et sous atmosphère d'air contenant 5 % de $CO_2$.

Apres les 5 j d'incubation, les cellules sont décollées à La trypsine et comptées avec l'appareil dénommé Coulter Counter.

Le produit testé est un extrait méthanolique brut de Prunella Vulgaris selon l'exemple 1, solubilisé dans du DMSO.

Ce produit est introduit dans le milieu de culture à différentes concentrations, de 1 $\delta$g à 10 $\delta$g (extrait sec) par millilitre de milieu de culture.

Les résultats obtenus sont répertoriés au tableau I, dans lequel sont indiqués le nombre moyen de cellules par boîte, après les 5 j d'incubation et, pour les boîtes traitées, l'activité A des produits testés, calculée à partir du nombre de cellules dans chaque catégorie de boîtes :

$$A = \frac{\text{boîtes traitées - boîtes témoins}}{\text{boîtes témoins}} \times 100$$

L'étude statistique a montré que les différences entre le nombre de cellules dans les boîtes traitées et les boîtes témoins, après incubation, étaient toutes significatives.

TABLEAU I

| Concentration extrait invention exemple 1 ($\mu$g/ml) | Nombre de cellules par boîte x $10^3$ | Activité A % | Significabilité |
|---|---|---|---|
| témoin DMSO | $90 \pm 13$ | 0 | |
| 1 | $134 \pm 23$ | + 49 | S |
| 2,5 | $173 \pm 2$ | + 92 | S |
| 10 | cytotoxique | | |

## Exemple 4

### Mise en évidence de l'inhibition de la phospholipase $A_2$ (ou $PLA_2$)

On mesure l'inhibition de la phospholipase $A_2$ qui intervient dans la production des médiateurs de l'allergie et de l'inflammation, notamment dans les réactions inflammatoires des allergies cutanées, selon le protocole décrit par H.W. Chang, I. Kudo, M Tomita et K. Inoue dans J. Biol. Chem., 1987, 102, 147-154.

On isole la phospholipase $A_2$ du fluide péritonéal de souris. La phospholipase $A_2$ hydrolyse en position 2 du glycérol une phosphatidyléthanolamine radio-marquée au carbone 14, libérant un acide gras qui sera dosé par scintillation liquide. Avec un inhibiteur de cette enzyme, l'hydrolyse sera moindre, et on dosera donc moins d'acide gras.

En pratique, on met la phospholipase $A_2$ plus l'extrait à tester dans un tampon Tris 0,1M, pH = 9, contenant 4 mM de $Ca^{2+}$, on incube 40 min à 37°C. Après ce temps de contact, on ajoute le substrat marqué et on incube de nouveau 40 min à 37°C pour permettre l'hydrolyse du substrat. On extrait alors, par une phase organique de n-heptane, l'acide gras que l'on compte en scintillation liquide. On détermine alors une concentration d'inhibition 50 ($CI_{50}$) pour laquelle 50 % de l'enzyme est inhibée.

Ainsi, avec l'extrait méthanolique de Brunelle selon l'exemple 1, on obtient une concentration d'inhibition 50 de 17 $\mu$g/ml, ce qui démontre une très bonne activité d'inhibition de la phospholipase $A_2$ grâce à cet extrait de Brunelle.

Le même test effectué avec un extrait aqueux de Brunelle permet d'obtenir une $CI_{50}$ de 28 $\delta$g/ml.

## Exemple 5

### Activité anti-inflammatoire d'extrait de Brunelle vis-à-vis de l'érythème provoqué par les ultraviolets

Pour ce faire, on utilise 41 cobayes Dunkin Hartley femelles pesant 400 à 450 g, répartis en 6 lots de 6 cobayes et un lot de 5 cobayes.

Les animaux sont stabulés pendant une semaine avant le début de l'expérimentation à 20° plus ou moins 1°C en température et 50 % plus ou moins 10 % d'hygrométrie et 12 h de lumière par jour. Les animaux sont soigneusement tondus la veille de l'expérience sur le dos à l'aide d'une tondeuse AESCULAP munie d'un peigne de 1/10 mm, puis rasés délicatement au niveau des deux flancs à l'aide d'un rasoir Braun. Ensuite, une crème dépilatoire est appliquée (environ 2 mm d'épaisseur), et on la laisse agir pendant 15 min.

La crème est retirée à l'aide d'une spatule en bois, puis les cobayes sont rincés à l'eau tiède et séchés.

Les cobayes reçoivent ensuite sur les deux flancs la même irradiation, les plages irradiées faisant 2 cm$^2$ de surface. Le flanc droit reçoit le produit à tester, le produit étant étalé au-delà des 2 cm$^2$, cela permettant d'observer une éventuelle rougeur due au produit et qui masquerait un effet antiphlogistique. Le flanc gauche reçoit le placebo.

L'application du placebo sur le flanc gauche permet de mesurer dans les mêmes conditions l'effet des UV sur les zones traitées par le produit, et sur celles non traitées. Cela permet également de voir une éventuelle action du placebo et de lire comparativement au placebo la zone où le produit a été appliqué.

L'application s'effectue immédiatement après l'irradiation, puis toutes les 2 h, jusqu'à 6 h après l'irradiation.

La quantité appliquée n'est pas mesurée mais étalée uniformément sur toute la plage (environ 1 mg).

Le dispositif d'irradiation utilisé est constitué d'une lampe au mercure Biotronic UV312-365 (Vilbert-Loumat). Le dispositif de mesure de l'énergie UV constitué d'une cellule photoélectrique, mesurant la puissance lumineuse des UV, est fixé sur un support au centre de la zone irradiée. Cette cellule est reliée à un radiomètre, sur lequel l'énergie désirée - ici de 2,16 J/cm$^2$ d'UVB (distance du cobaye à la lampe = 20 cm) est programmée. La lampe s'éteint automatiquement une fois que la totalité de l'énergie est émise.

Cette irradiation entraîne un érythème égal à 3 plus ou moins 0,5 (voir échelle) à 6 h.

Les animaux sont anesthésiés 20 min avant l'irradiation au pentobarbital sodique à la dose de 0,5 ml/kg par voie intra-péritonéale.

Six heures après l'irradiation, l'érythème de chaque plage est évalué macroscopiquement selon l'échelle suivante :

0      pas d'érythème
1      érythème léger mais visible
2      érythème bien visible
3      érythème fort

Un cobaye à 1 selon la notation ci-dessus, un cobaye à 2 et un cobaye à 3 sont retenus après les avoir bien évalués comme tels, et sont pris en référence étalon pour permettre de noter en demi. Un cobaye n'étant pas évalué à 1 (référence étalon) et n'étant pas évalué à 2 sera noté 1,5.

Les résultats sont reportés au tableau II.

Les résultats de la différence (UV + placebo) - (UV + produit) sont reportés dans le tableau II bis.

A partir des résultats obtenus, le pourcentage d'activité est calculé selon la formule suivante :

$$\text{pourcentage d'activité} = \frac{\overline{X} \text{ (témoin UV + placebo) - } \overline{X} \text{ (UV produit)}}{\overline{X} \text{ (témoin UV + placebo)}} \times 100$$

TABLEAU II

| Notation | Extrait aqueux Brunelle dans gel aqueux | | Extrait méthanolique exemple 1 dans gel éthanolique | | Indométacine dans l'hydrocérine | |
|---|---|---|---|---|---|---|
| | placebo | produit | placebo | produit | placebo | produit |
| 1 | 3 | 1,5 | 3 | 1,5 | 3 | 0,5 |
| 2 | 2,5 | 2,5 | 2,5 | 1 | 3 | 1 |
| 3 | 2,5 | 1,5 | 2,5 | 1,5 | 3 | 0 |
| 4 | 2 | 1 | 3 | 1,5 | 3 | 0 |
| 5 | 2 | 1,5 | 2,5 | 1,5 | 2,5 | 0,5 |
| 6 | 2,5 | 1,5 | 2,5 | 1 | | |
| moyenne | 2,41 | 1,58 | 2,66 | 1,33 | 2,9 | 0,4 |
| écart type | 0,37 | 0,49 | 0,25 | 0,26 | 0,22 | 0,41 |
| Protection | 34,4 % | | 50 % | | 86,2 % | |
| t de Student | 3,95 >** | | 12,64 >*** | | 11,18 >*** | |
| 1 | 1,5 | | 1,5 | | 2,5 | |
| 2 | 0 | | 1,5 | | 2 | |
| 3 | 1 | | 1 | | 3 | |
| 4 | 1 | | 1,5 | | 3 | |
| 5 | 0,5 | | 1 | | 2 | |
| 6 | 1 | | 1,5 | | | |
| Moyenne | 0,83 | | 1,33 | | 2,5 | |
| Ecart type | 0,52 | | 0,26 | | 0,5 | |

> ** significatiff à 1 %
> *** significatiff à 0,1 %

On constate ainsi que les extraits de Brunelle selon la présente invention présentent une activité de 34,4 % ou de 50 %, qui est à comparer avec l'activité de l'indométacine de 86,2 % agissant en tant que témoin positif.

On observera que les produits de l'invention apportent ainsi une bonne activité antiphlogistique dans les conditions expérimentales, évitant ainsi de faire appel à des produits tels que l'indométacine qui étaient auparavant nécessaires pour obtenir une activité satisfaisante.

Les extraits selon l'invention testés sont à 0,2 % en poids dans le gel. L'indométacine est à 1 % dans l'hydrocérine.

Exemple 6

Activité sur la différenciation des kératinocytes

Lors du vieillissement un certain nombre de modifications apparaissent au niveau des kératinocytes. Tout d'abord, il y a lieu de préciser que les kératinocytes ne sont pas une population totalement homogène, mais plutôt une mosaïque de cellules. Au niveau ultrastructural, on distingue d'après la littérature :

a) Le vieillissement intrinsèque

La couche cornée est épaissie, les cornéocytes moins adhérents, il semble ne pas y avoir de modification quantitative et qualitative très sensible des tonofilaments, des grains de kératohyaline et des organelles cellulaires.

b) <u>Vieillissement actinique</u>

Les espaces intercellulaires sont élargis, il y a présence de vacuoles intracytoplasmiques, les tonofilaments sont plus courts et répartis en mailles, les desmosomes sont altérés en nombre variable, il y a une diminution des projections microvillositaires.

Pour la présente étude, on a effectué un prélèvement de peau lors d'une opération de chirurgie esthétique de type "lifting" sur le visage d'une femme de 42 ans.

On a réalisé de manière classique une culture de kératinocytes isolés à partir de ce prélèvement dans six boîtes de Petri. Trois d'entre elles sont traitées avec l'extrait méthanolique de Brunelle de l'exemple 1 à une dose non cytotoxique de 0,25 µg/ml, Les trois autres ne sont pas traitées.

Les cultures de kératinocytes, respectivement traitées à l'extrait de Brunelle selon l'exemple 1 et non traitées (témoin), sont incluses dans une résine, puis on réalise une coupe de chacune d'elles que l'on observe au microscope électronique à transmission. Les résultats de cette observation sont répertoriés au tableau III suivant.

TABLEAU III

|  | Témoin | Extrait de Brunelle, exemple 1 |
|---|---|---|
| Couche supérieure | signes de dégénération pas d'enveloppe cornée pas de grains de kératohyaline, ou ceux-ci sont importants pour la kératinisation | couche cornée ++ beaucoup de grains de kératohyaline moins d'organelles 2 à 3 couches de cornification |
| Couche intermédiaire | quelques tonofilaments grands espaces intercellulaires quelques desmosomes | desmosomes très nombreux et très différenciés beaucoup de tonofilaments |
| Couche basale | quelques hémidesmosomes | nombreux desmosomes |

On constate du tableau III ci-dessus qu'avec l'extrait de Brunelle selon la présente invention, on obtient une culture très différenciée, ce qui démontre que l'extrait de Brunelle présente une activité antivieillissement et régulatrice de la différenciation des kératinocytes, de façon hautement significative.

Egalement, en raison de l'effet de normalisation de la différenciation des kératinocytes, les compositions selon l'invention permettent de maintenir un bon état de l'épiderme de la peau "normale", notamment par le maintien de sa souplesse et de son rôle fonctionnel, en particulier son rôle de barrière de protection.

Les compositions de l'invention peuvent être utilisées pour la prévention ou le traitement des peaux sèches, notamment des peaux ichtyosiques.

L'invention peut encore être utilisée pour le traitement du psoriasis.

Il est à noter en effet que les phénomènes des peaux sèches et du psoriasis sont accompagnés d'un trouble de différenciation des kératinocytes. En particulier, les kératinocytes psoriatiques sont pauvrement développés et immatures, le nombre et la taille des tonofilaments sont altérés, certaines cellules de la couche cornée contiennent encore des organelles, voire un noyau, ce qui montre que la différenciation ne s'est pas faite correctement.

Dans le cas des peaux sèches, en particulier de l'ichtyose, la différenciation des kératinocytes est imparfaite, s'accompagnant de malformations des grains de kératohyaline et des desmosomes. L'épiderme présente une kératinisation anormale, ce qui entraîne une perturbation des propriétés de barrière et une perte d'élasticité.

<u>Exemple 7</u>

<u>Mise en évidence de l'activité des produits de l'invention sur le système pilaire</u>

Le test repose sur l'étude de l'activité des produits selon l'invention sur le cycle pilaire de rats Sprague Dawley tous âges de 23 j (j = jour). A cet âge, les cycles pilaires de tous les animaux sont encore synchrones.

Plus particulièrement, on cherche à mettre en évidence l'action des produits à tester sur la prolongation de la phase de croissance des poils, dite "phase anagène".

Pour cela, on opère de la façon suivante. Tous les rats sont tondus à 24 j sur la partie inférieure du dos, au niveau des flancs, de manière à ne laisser qu'une faible longueur du poil, juste nécessaire pour permettre les épilations ultérieures.

On applique ensuite à partir du 25e j (âge des rats) jusqu'au 65e j, 6 j sur 7, les produits à tester, à une dose évoluant avec le poids des animaux. Cette dose est de 0,5 ml au 25e j, et atteint 2 ml au 65e j.

A partir du 28e j et à intervalle de temps sensiblement régulier (environ tous les 3 j), on prélève à l'aide d'une pince à épiler une touffe de poils sur le côté gauche de l'animal au niveau du flanc. On observe à fort grossissement les bulbes de 10 poils pris au hasard dans cette touffe, et on compte le nombre de poils en phase anagène reconnaissables à la forme caractéristique du bulbe. On détermine ainsi par lot de 10 animaux le pourcentage de poils en phase anagène (phase de croissance), en fonction du temps.

L'étude est effectuée sur 30 rats répartis en 3 lots de 10 animaux. Le premier lot reçoit une préparation selon l'invention de composition suivante (composition "I") :

| - extrait de Brunelle selon l'exemple 1 | 0,1 g |
|---|---|
| - cépharanthine | 0,1 g |
| - propylèneglycol | 20,0 g |
| - eau distillée | 15,0 g |
| - alcool éthylique à 95 % qsp | 100,0 g |

Le second ne reçoit que l'excipient précité. Le troisième lot est le lot témoin. Il ne reçoit aucun produit.

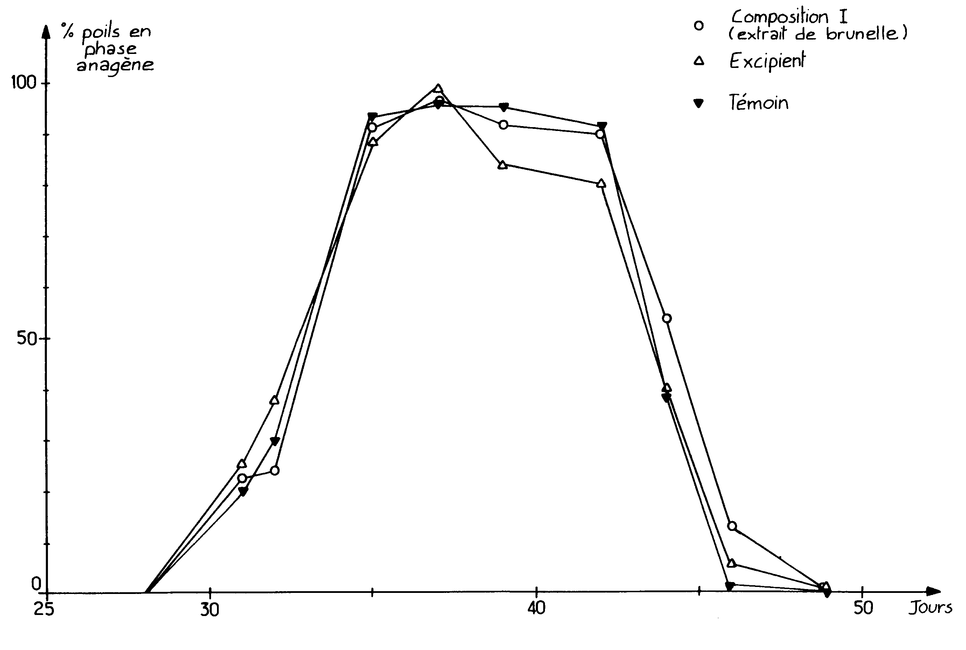

Les résultats de cette étude trichocinétique (trichogramme en fonction du temps) sont reportés, en valeurs moyennes, au tableau IV et représentés sur la figure unique annexée.

TABLEAU IV

| | Pourcentage poils anagènes | | | | | | | | | |
|---|---|---|---|---|---|---|---|---|---|---|
| | 28 j | 31 j | 32 j | 35 j | 37 j | 39 j | 42 j | 44 j | 46 j | 49 j |
| Composition "I" (invention) | 0 | 23 | 24 | 92 | 96 | 92 | 90 | 53 | 13 | 0 |
| Excipient seul | 0 | 26 | 38 | 88 | 98 | 83 | 80 | 40 | 5 | 0 |
| Témoin | 0 | 21 | 30 | 93 | 96 | 95 | 91 | 38 | 1 | 0 |

Sur cette figure, on a représenté en ordonnée le pourcentage de poils en phase anagène, et en abcisse l'âge des animaux exprimé en jours.

La courbe joignant les ronds correspond aux résultats obtenus avec la préparation selon l'invention (composition "I"), la courbe joignant les triangles avec pointe en haut est obtenue avec l'excipient et enfin, la courbe joignant les triangles avec pointe en bas est obtenue avec le lot témoin ne recevant aucun produit.

On observe que la phase anagène se prolonge plus long-temps chez les rats traités par le produit selon l'invention, notamment par comparaison avec les rats traités par l'excipient seulement. Ceci est particulièrement net à partir du 37e j.

Ainsi, il est clair que les extraits de Brunelle selon l'invention, par rallongement de la durée de la phase anagène, retardent très nettement la chute des cheveux et favorisent la repousse.

On donnera ci-après divers exemples de formulation de compositions cosmétiques ou pharmaceutiques, favorisant la repousse des cheveux et/ou retardant leur chute et/ou favorisant ou régulant le renouvellement ou la croissance de l'épiderme.

Exemple 8

Gel régulateur de l'épiderme

On dissout dans 49,2 g d'eau distillée 0,2 g d'extrait de Brunelle obtenu par extraction avec de l'eau selon une procédure similaire à celle décrite à l'exemple 1, puis l'on gélifie cette solution en ajoutant 50 g d'un gel neutralisé de Carbopol 940 R à 3 %.

On peut appliquer ce gel deux fois par jour par cure de 4 mois.

Exemple 9

Lotion anti-chute des follicules pileux, en particulier des cheveux

On prépare la composition suivante :

| - extrait de Brunelle selon l'exemple 1 | 0,1 g |
|---|---|
| - panthénol | 0,1 g |
| - hydrolysat de kératine | 0,3 g |
| - parfum | 0,1 g |
| - alcool à 40 % qsp | 100 g |

Exemple 10

Gel coiffant anti-chute

On prépare la composition suivante de manière bien connue à l'homme de l'art :

| - extrait de Brunelle selon l'exemple 1 | 0,2 g |
|---|---|
| - gel de Carbopol 940® 1,50 % neutralisé | 45 g |
| - Phytantriol® | 0,1 g |
| - Crémophor RH40® | 0,5 g |
| - complexe protéine-zinc | 0,1 g |
| - conservateur | 0,05 g |
| - excipient aqueux parfumé qsp | 100 g |

Le gel obtenu est appliqué matin et soir sur les zones de chute des follicules pileux pendant 6 mois.

Exemple 11

Gel traitant contre l'alopécie séborrhéique

On prépare la composition suivante :

| - extrait de Brunelle selon l'exemple 1 | 0,2 g |
|---|---|
| - Crémophor RH40® | 0,5 g |
| - parfum | 0,1 g |
| - complexe protéine-zinc | 0,05 g |
| - gel Carbopol 940® 1,50 % | 45 g |
| - excipient aqueux qsp | 100 g |

On peut appliquer ce gel matin et soir sur les cheveux pendant 6 mois.

Exemple 12

Crème traitante contre la sénescence cutanée

On prépare une crème à 0,2 % d'extrait de Brunelle de la manière suivante :

| Composition A (pourcentage en poids) | |
| --- | --- |
| - Tefosse 1500$^{®}$ de chez Gattefossé | 7,00 |
| - alcool cétylique | 2,00 |
| - Primol 352$^{®}$ de chez ESSO | 25,00 |
| Composition B (pourcentage en poids) | |
| - eau déminéralisée | 54,40 |
| - Carbopol 940$^{®}$ de chez Polyplastic | 0,30 |
| - triéthanolamine | 0,30 |
| Composition C (pourcentage en poids) | |
| - Germaben II$^{®}$ de chez Seppic | 0,80 |
| Composition D (pourcentage en poids) | |
| - eau déminéralisée | 10,00 |
| - extrait aqueux de Brunelle (extrait sec) | 0,20 |

On chauffe la composition A à 75°C, puis on disperse le Carbopol 940$^{®}$ dans l'eau de la composition B. On neutralise le gel avec de la triéthanolamine. On chauffe la solution B à 75°C. On réalise une émulsion de la composition A dans la composition B à 75°C. On refroidit sous agitation jusqu'à 40°C.

On ajoute ensuite la composition C. On réalise le prémélange des composants de la composition D, puis on ajoute la composition D à la formule. On homogénéise et on refroidit jusqu'à la température ambiante en obtenant ainsi une crème.

On peut appliquer cette crème deux fois par jour par cure de 6 mois pour obtenir une peau souple régénérée.

Exemple 13

Gel pour le traitement du psoriasis

| | |
| --- | --- |
| extrait de Brunelle selon l'exemple 1 | 0,15 g |
| gel Carbopol 980$^{®}$ à 1,5 % | 48, g |
| excipient aqueux qsp | 100, g |

Utiliser en applications locales pendant 12 semaines, 2 fois par jour.

Exemple 14

Emulsion pour peaux sèches

| | |
|---|---|
| extrait de Brunelle selon l'exemple 1 | 0,05 g |
| acide hyaluronique | 1, g |
| glycérol | 1, g |
| excipient pour émulsion huile dans eau, qsp | 100, g |

Appliquer cette crème pendant 6 semaines sur les zones sèches de la peau.

Exemple 15

Emulsion pour peaux sèches

| | |
|---|---|
| extrait de Brunelle selon l'exemple 1 | 0,15 g |
| excipient pour émulsion eau dans huile, qsp | 100, g |

Utiliser en applications locales 2 fois par jour pendant 4 semaines sur les zones ichtyosées de la peau.

Naturellement, l'invention comprend tous les moyens constituant des équivalents techniques des moyens décrits ainsi que leurs diverses combinaisons. En particulier, l'invention couvre non seulement le traitement cosmétique ou pharmaceutique de la peau mais aussi celui des phanères tels que les cheveux et les ongles.

**Revendications**

1. Utilisation d'un extrait de Brunelle ou d'au moins une saponine de Brunelle pour la préparation d'une composition pharmaceutique, notamment dermatologique, présentant une activité anti-radicalaire, une activité anti-inflammatoire, notamment dans les inflammations provoquées par le rayonnement ultraviolet, régulant le renouvellement et la différenciation des kératinocytes, permettant d'améliorer ou de maintenir un bon état fonctionnel de la peau ou des phanères, de prévenir ou de traiter des peaux sèches, notamment des peaux ichtyosiques, ou de traiter le psoriasis.

2. Utilisation d'un extrait de Brunelle ou d'au moins une saponine de Brunelle, comme agent cosmétique destiné à améliorer ou maintenir un bon état fonctionnel de la peau ou des phanères, à lutter contre les effets du vieillissement de l'épiderme ou des phanères, à prévenir, à traiter les peaux sèches, notamment les peaux ichtyosiques, ou à lutter contre les inflammations, notamment l'érythème provoqué par les rayonnements ultraviolets.

3. Utilisation d'un extrait de Brunelle ou d'au moins une saponine de Brunelle pour favoriser ou réguler la différenciation et le renouvellement des cellules de l'épiderme en culture.

4. Utilisation selon la revendication 1, 2, ou 3, caractérisée en ce que la Brunelle précitée est la Prunella Vulgaris L. ou Brunelle commune.

5. Utilisation selon l'une des revendications 1 à 4, caractérisée en ce que l'extrait de Brunelle précité ou la saponine de Brunelle précitée est obtenu par extraction à partir de la plante entière, ou de préférence à partir des parties aériennes de Brunelle.

6. Utilisation selon l'une des revendications 1 à 5, caractérisée en ce que l'extrait de Brunelle précité est obtenu grâce à une extraction primaire de la matière sèche, la matière sèche, constituée de préférence de parties aériennes de Brunelle, au moyen d'un solvant choisi parmi le groupe constitué par : l'eau, les alcools, comportant de préférence de 1 à 4 atomes de carbone, les solvants chlorés comportant de préférence de 1 à 2 atomes de carbone, les esters organiques comportant de préférence de 3 à 6 atomes de carbone ; ou d'un solvant mixte à base d'un mélange quelconque des solvants précités.

7. Utilisation selon la revendication 6, caractérisée en ce que le solvant d'extraction primaire précité est choisi parmi le groupe constitué par : l'eau, le méthanol, l'éthanol, un mélange méthanol-eau ou un mélange éthanol-eau, le chloroforme, le dichlorométhane ; de préférence ce solvant est choisi parmi l'eau ou le méthanol.

8. Utilisation selon l'une des revendications 1 à 7, caractérisée en ce qu'elle est relative à un mélange de saponines de Brunelle.

9. Utilisation selon la revendication 8, caractérisée en ce que l'on obtient un mélange de saponines de Brunelle à partir de l'extrait de Brunelle précité.

10. Utilisation selon l'une des revendications 1 à 9, caractérisée en ce que la concentration en extrait de Brunelle ou en saponine de Brunelle est comprise entre 0,001 % et 5 % en poids de la composition totale.

11. Utilisation selon l'une des revendications 1 à 10, caractérisée en ce que la composition comprend en outre au moins une autre substance active, en une concentration efficace, choisie parmi les xanthines, les vitamines, en particulier les vitamines A, B et E ou leurs dérivés par exemple les esters de vitamine A, la tyrosine ou ses dérivés comme par exemple le tyrosinate de glucose, la malyl tyrosine, la quinine ou ses dérivés, les rubéfiants tels que le nicotinate de méthyle, un surnageant de culture de fibroblastes de papilles, des hydrolysats de kératine, des oligo-éléments tels que zinc, sélénium, cuivre, un alcaloïde bisbenzylisoquinoléique tel que l'oxyacanthine ou la cépharanthine, des inhibiteurs de 5-$\alpha$-réductase tels que : progestérone, acétate de cyprotérone, minoxidil, acide azélaïque et ses dérivés, 1,diméthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

12. Utilisation selon l'une des revendications 1 à 11, caractérisée en ce que la composition contient en outre des phases lamellaires lipidiques hydratées ou des liposomes incorporant ou non l'extrait de Brunelle ou la saponine de Brunelle précité.

13. Composition cosmétique, ou pharmaceutique, notamment dermatologique, de préférence topique, caractérisée en ce qu'elle comprend à titre d'ingrédient actif une quantité cosmétiquement ou pharmaceutiquement efficace d'un extrait de Brunelle ou d'au moins une saponine de Brunelle, ainsi que des phases lamellaires lipidiques hydratées ou des liposomes incorporant ou non l'extrait de Brunelle ou une saponine de Brunelle, éventuellement dans un excipient cosmétiquement ou pharmaceutiquement acceptable.

14. Composition selon la revendication 13, caractérisée en ce que la concentration d'extrait de Brunelle ou de saponine de Brunelle est comprise entre 0,001 % et 5 % en poids de la composition totale.

15. Composition selon la revendication 13 ou 14, caractérisée en ce qu'elle comprend en outre au moins une autre substance active, en une concentration efficace, choisie parmi les xanthines, les vitamines, en particulier les vitamines A, B et E ou leurs dérivés par exemple les esters de vitamine A, la tyrosine ou ses dérivés comme par exemple le tyrosinate de glucose, la malyl tyrosine, la quinine ou ses dérivés, les rubéfiants tels que le nicotinate de méthyle, un surnageant de culture de fibroblastes de papilles, des hydrolysats de kératine, des oligo-éléments tels que zinc, sélénium, cuivre, un alcaloïde bisbenzylisoquinoléique tel que l'oxyacanthine ou la cépharanthine, des inhibiteurs de 5-$\alpha$-réductase tels que : progestérone, acétate de cyprotérone, minoxidil, acide azélaïque et ses dérivés, 1,diméthyl-4-azastéroïde, en particulier la 17-$\beta$-N,N-diéthylcarbamoyl-4-méthyl-4-aza-5-$\alpha$-androstan-3-one, ou encore un extrait de Serenoa repens.

16. Composition selon l'une des revendications 13 à 15, caractérisée en ce que la Brunelle précitée est la Prunella Vulgaris L. ou Brunelle commune.

17. Composition selon l'une des revendications 13 à 16, caractérisée en ce que l'extrait de Brunelle précité ou la saponine de Brunelle précitée est obtenu par extraction à partir de la plante entière ou de préférence à partir des parties aériennes de Brunelle.

18. Composition selon l'une des revendications 13 à 17, caractérisée en ce que l'extrait de Brunelle précité est obtenu grâce à une extraction primaire de la matière sèche, constituée de préférence de parties aériennes de Brunelle, au moyen d'un solvant choisi parmi le groupe constitué par: l'eau, les alcools, comportant de préférence de 1 à 4 atomes de carbone, les solvants chlorés comportant de préférence de 1 à 2 atomes de carbone, les esters organiques comportant de préférence de 3 à 6 atomes de carbone ; ou d'un solvant mixte à base d'un mélange quelconque des solvants précités.

19. Composition selon la revendication 18, caractérisée en ce que le solvant d'extraction primaire précité est choisi parmi le groupe constitué par : l'eau, le méthanol, l'éthanol, un mélange méthanol-eau ou un mélange éthanol-eau, le chloroforme, le dichlorométhane ; de préférence ce solvant est choisi parmi l'eau ou le méthanol.

20. Méthode de traitement cosmétique pour lutter contre les effets du veillissement sur l'épiderme ou les phanères, pour lutter contre les inflammations, notamment l'érythème provoqué par les rayonnements ultraviolets, pour améliorer ou maintenir un bon état fonctionnel de la peau ou des phanères, pour prévenir ou traiter les peaux sèches, ou pour traiter les désordres causés par l'action des radicaux libres, caractérisée en ce qu'elle comprend l'application topique, en une quantité efficace pour réaliser ledit effet recherché, d'au moins un extrait de Brunelle ou d'au moins une saponine de Brunelle, éventuellement incorporé dans un excipient cosmétiquement acceptable.

21. Méthode selon la revendication 20, caractérisée en ce que l'extrait de Brunelle ou d'au moins une saponine de Brunelle est tel(le) que défini(e) à l'une quelconque des revendications 4 à 12.

## Claims

1. Use of an extract of prunella or at least one prunella saponin for the preparation of a pharmaceutical composition, especially dermatological composition, having an anti-free-radical activity, an anti-inflammatory activity, especially in inflammations caused by ultraviolet radiation, said composition regulating the renewal and differentiation of the keratinocytes, for improving the functional condition of the skin or of the superficial body growths or keeping them in a good functional condition, for preventing or treating dry skin, especially ichthyotic skin, or for treating psoriasis.

2. Use of an extract of prunella or at least one prunella saponin, as a cosmetic agent intended to improve the functional condition of the skin or of the superficial body growths or keep them in a good functional condition, to combate the effects of ageing of the epidermis or of the superficial body growths, to prevent, to treat dry skin, especially ichthyotic skin, or to combate inflammations, especially erythema caused by ultraviolet radiation.

3. Use of an extract of prunella or at least one prunella saponin for promoting or regulating the differentiation and the renewal of the cells of the epidermis in culture.

4. Use according to claim 1, 2 or 3, characterised in that the above-mentioned prunella is Prunella vulgaris L. or common prunella.

5. Use according to one of claims 1 to 4, characterised in that the above-mentioned extract of prunella or the above-mentioned prunella saponin is obtained by extraction from the whole plant or, preferably, from the aerial parts of prunella.

6. Use according to one of claims 1 to 5, characterised in that the above-mentioned extract of prunella is obtained by carrying out a primary extraction of the dry matter, said dry matter preferably consisting of aerial parts of prunella, with a solvent selected from the group consisting of water, alcohols preferably containing from 1 to 4 carbon atoms, chlorinated solvents preferably containing 1 or 2 carbon atoms, organic esters preferably containing from 3 to 6 carbon atoms, or a mixed solvent based on any mixture of the above-mentioned solvents.

7. Use according to claim 6, characterised in that the above-mentioned primary extraction solvent is selected from the group consisting of water, methanol, ethanol, a methanol/water mixture or an ethanol/water mixture, chloroform and dichloromethane, this solvent preferably being selected from water or methanol.

8. Use according to one of claims 1 to 7, characterised in that it relates to a mixture of prunella saponins.

9. Use according to claim 8, characterised in that a mixture of prunella saponins is obtained from the above-mentioned extract of prunella.

10. Use according to one of claims 1 to 9, characterised in that the concentration of extract of prunella or prunella saponin is between 0.001% and 5% by weight of the total composition.

11. Use according to one of claims 1 to 10, characterised in that the composition also comprises an effective concentration of at least one other active substance selected from xanthines, vitamins, in particular vitamins A, B and E, or derivatives thereof, for example vitamin A esters, tyrosine, or derivatives thereof, for example glucose tyrosinate and malyltyrosine, quinine or derivatives thereof, rubefacients such as methyl nicotinate, a papilla fibroblast culture supernatant, keratin hydrolyzates, trace elements such as zinc, selenium and copper, a bisbenzylisoquinoline alkaloid such as oxyacanthine or cepharanthine, 5-$\alpha$-reductase inhibitors such as progesterone, cyproterone acetate and minoxidil, azelaic acid and derivatives thereof, a 1,4-dimethyl-4-azasteroid, in particular 17-$\beta$-N,N-diethylcarbarnoyl-4-methyl-4-aza-5-$\alpha$-androstan-3-one, or else an extract of Serenoa repens.

12. Use according to one of claims 1 to 11, characterised in that the composition also contains hydrated lipidic lamellar phases or liposomes, which may or may not incorporate the extract of prunella or the prunella saponin mentioned above.

13. A cosmetic or pharmaceutical composition, especially dermatological composition, preferably a topical composition, characterised in that it comprises, as the active ingredient, a cosmetically or pharmaceutically effective amount of an extract of prunella or at least one prunella saponin, as well as hydrated lipidic lamellar phases or liposomes, which may or may not incorporate the extract of prunella or a prunella saponin, if appropriate in a cosmetically or pharmaceutically acceptable excipient.

14. A composition according to claim 13, characterised in that the concentration of extract of prunella or prunella saponin is between 0.001% and 5% by weight of the total composition.

15. A composition according to claim 13 or 14, characterised in that it also comprises an effective concentration of at least one other active substance selected from xanthines, vitamins, in particular vitamins A, B and E, or derivatives thereof, for example vitamin A esters, tyrosine or derivatives thereof, for example glucose tyrosinate and malyltyrosine, quinine or derivatives thereof, rubefacients such as methyl nicotinate, a papilla fibroblast culture supernatant, keratin hydrolyzates, trace elements such as zinc, selenium and copper, a bisbenzylisoquinoline alkaloid such as oxyacanthine or cepharanthine, 5-$\alpha$-reductase inhibitors such as progesterone, cyproterone acetate and minoxidil, azelaic acid and derivatives thereof, a 1,4-dimethyl-4-azasteroid, in particular 17-$\beta$-N,N-diethylcarbamoyl-4-methyl-4-aza-5-$\alpha$-androstan-3-one, or else an extract of Serenoa repens.

16. A composition according to one of claims 13 to 15, characterised in that the above-mentioned prunella is Prunella vulgaris L. or common prunella.

17. A composition according to one of claims 13 to 16, characterised in that the above-mentioned extract of prunella or the above-mentioned prunella saponin is obtained by extraction from the whole plant or, preferably, from the aerial parts of prunella.

18. A composition according to one of claims 13 to 17, characterised in that the above-mentioned extract of prunella is obtained by carrying out a primary extraction of the dry matter, said dry matter preferably consisting of aerial parts of prunella, with a solvent selected from the group consisting of water, alcohols preferably containing from 1 to 4 carbon atoms, chlorinated solvents preferably containing 1 or 2 carbon atoms, organic esters preferably containing from 3 to 6 carbon atoms, or a mixed solvent based on any mixture of the above-mentioned solvents.

19. A composition according to claim 18, characterised in that the above-mentioned primary extraction solvent is selected from the group consisting of water, methanol, ethanol, a methanol/water mixture or an ethanol/water mixture, chloroform and dichloromethane, this solvent preferably being selected from water or methanol.

20. A cosmetic treatment method for combating the effects of ageing on the epidermis or the superficial body growths, for combating inflammations, especially erythema caused by ultraviolet radiation, for improving the functional condition of the skin or of the superficial body growths or keeping them in good functional condition, for preventing or treating dry skin or for treating disorders caused by the action of free radicals, said method being characterised in that it comprises the topical application, in an effective amount for achieving said desired effect, of at least one extract of prunella or at least one prunella saponin, if appropriate incorporated in a cosmetically acceptable excipient.

21. A method according to claim 20, characterised in that the extract of prunella or at least one prunella saponin is as defined in any one of claims 4 to 12.

**Patentansprüche**

1. Verwendung eines Extrakts von Braunelle oder zumindest einem Saponin von Braunelle bei der Herstellung einer pharmazeutischen, insbesondere dermatologischen Zusammensetzung mit einer Anti-Radikal-Wirksamkeit, einer entzündungshemmenden Wirksamkeit, insbesondere bei durch Ultraviolett-Strahlung hervorgerufenen Entzündungen, welche die Erneuerung und die Differenzierung von Keratinozyten reguliert, zur Verbesserung oder Aufrechterhaltung eines guten funktionellen Zustands der Haut oder der Hautanhangsgebilde, zur Prävention oder zur Behandlung von trockener Haut, insbesondere sich schuppender Haut, oder zur Behandlung von Psoriasis.

2. Verwendung eines Extrakts von Braunelle oder zumindest einem Saponin von Braunelle als kosmetisches Mittel zur Verbesserung oder Aufrechterhaltung eines guten funktionellen Zustands der Haut oder der Hautanhangsgebilde, zur Bekämpfung von Effekten der Alterung der Epidermis oder Hautanhangsgebilde, zur Prävention und zur Behandlung von trockener Haut, insbesondere sich schuppender Haut, oder zur Bekämpfung von Entzündungen, insbesondere Erythem, das durch Ultraviolett-Strahlung hervorgerufen wird.

3. Verwendung eines Extrakts von Braunelle oder zumindest einem Saponin von Braunelle zur Förderung oder Regulierung der Differenzierung und der Erneuerung von Zellen der Epidermis in Kultur.

4. Verwendung nach Anspruch 1, 2 oder 3, dadurch gekennzeichnet, daß die Braunelle Prunella Vulgaris L. oder Gemeine Braunelle ist.

5. Verwendung nach einem der Ansprüche 1 bis 4, dadurch gekennzeichnet, daß der Extrakt von Braunelle oder dem Saponin von Braunelle durch Extraktion aus der ganzen Pflanze oder vorzugsweise aus den an der Luft befindlichen Teilen von Braunelle erhalten wird.

6. Verwendung nach einem der Ansprüche 1 bis 5, dadurch gekennzeichnet, daß der Braunelle-Extrakt aus einer primären Extraktion von Trockenmaterial, wobei das Trockenmaterial vorzugsweise aus an der Luft befindlichen Teilen von Braunelle besteht, mit einem Lösungsmittel, ausgewählt aus der Gruppe bestehend aus Wasser, Alkoholen mit vorzugsweise 1 bis 4 Kohlenstoffatomen, chlorierten Lösungsmitteln mit vorzugsweise 1 bis 2 Kohlenstoffatomen, organischen Estern mit vorzugsweise 3 bis 6 Kohlenstoffatomen, oder mit einem gemischten Lösungsmittel auf der Basis einer beliebigen Mischung der angegebenen Lösungsmittel erhalten wird.

7. Verwendung nach Anspruch 6, dadurch gekennzeichnet, daß das Lösungsmittel der primären Extraktion ausgewählt wird aus der Gruppe bestehend aus Wasser, Methanol, Ethanol, einer Methanol-Wasser-Mischung oder einer Ethanol-Wasser-Mischung, Chloroform, Dichlormethan, wobei dieses Lösungsmittel vorzugsweise aus Wasser oder Methanol ausgewählt wird.

8. Verwendung nach einem der Ansprüche 1 bis 7, dadurch gekennzeichnet, daß sie eine Mischung von Saponinen von Braunelle betrifft.

9. Verwendung nach Anspruch 8, dadurch gekennzeichnet, daß eine Mischung von Saponinen von Braunelle aus dem Braunelle-Extrakt erhalten wird.

10. Verwendung nach einem der Ansprüche 1 bis 9, dadurch gekennzeichnet, daß die Konzentration des Extrakts von Braunelle oder von Saponin von Braunelle zwischen 0,001 % und 5 %, bezogen auf die Masse der Gesamtzusammensetzung, beträgt.

11. Verwendung nach einem der Ansprüche 1 bis 10, dadurch gekennzeichnet, daß die Zusammensetzung außerdem zumindest eine andere aktive Substanz in einer wirksamen Konzentration umfaßt, ausgewählt aus Xanthinen, Vitaminen, insbesondere den Vitaminen A, B und E, oder ihren Derivaten, beispielsweise Estern von Vitamin A, Tyrosin oder seinen Derivaten, wie beispielsweise Glucosetyrosinat, Malyltyrosin, Chinin oder seinen Derivaten, Rubefazientien, wie Methylnicotinat, einem Papillenfibroblasten-Kulturüberstand, Keratinhydrolysaten, Spurenelementen, wie Zink, Selen, Kupfer, einem Bisbenzylisochinolin-Alkaloid, wie Oxyacanthin oder Cepharanthin, 5-$\alpha$-Reduktase-Inhibitoren, wie Progesteron, Cyproteronacetat, Minoxidil , Azelainsäure und ihren Derivaten, 1-Dimethyl-4-azasteroid, insbesondere 17-$\beta$-N,N-Diethylcarbamoyl-4-methyl-4-aza-5-$\alpha$-androstan-3-on, oder auch einen Extrakt von Serenoa repens.

12. Verwendung nach einem der Ansprüche 1 bis 11, dadurch gekennzeichnet, daß die Zusammensetzung außerdem hydratisierte lamellare Lipidphasen oder Liposomen, die gegebenenfalls einen Extrakt von Braunelle oder einem Saponin von Braunelle einschließen, enthält.

13. Kosmetische oder pharmazeutische, insbesondere dermatologische, vorzugsweise topische Zusammensetzung, dadurch gekennzeichnet, daß sie als aktiven Bestandteil eine kosmetisch oder pharmazeutisch wirksame Menge eines Extrakts von Braunelle oder zumindest einem Saponin von Braunelle sowie lamellare Lipidphasen oder Liposomen, die gegebenenfalls einen Extrakt von Braunelle oder einem Saponin von Braunelle einschließen, gegebenenfalls in einem kosmetisch oder pharmazeutisch annehmbaren Exzipienten umfaßt.

14. Zusammensetzung nach Anspruch 13, dadurch gekennzeichnet, daß die Konzentration des Extrakts von Braunelle oder einem Saponin von Braunelle zwischen 0,001 % und 5 %, bezogen auf die Masse der Gesamtzusammensetzung, beträgt.

15. Zusammensetzung nach Anspruch 13 oder 14, dadurch gekennzeichnet, daß sie außerdem zumindest eine andere aktive Substanz in einer wirksamen Konzentration umfaßt, ausgewählt aus Xanthinen, Vitaminen, insbesondere den Vitaminen A, B und E, oder ihren Derivaten, beispielsweise Estern von Vitamin A, Tyrosin oder seinen Derivaten, wie beispielsweise Glucosetyrosinat, Malyltyrosin, Chinin oder seinen Derivaten, Rubefazientien, wie Methylnicotinat, einem Papillenfibroblasten-Kulturüberstand, Keratinhydrolysaten, Spurenelementen, wie Zink, Selen, Kupfer, einem Bisbenzylisochinolin-Alkaloid, wie Oxyacanthin oder Cepharanthin, 5-$\alpha$-Reduktase-Inhibitoren, wie Progesteron, Cyproteronacetat, Minoxidil , Azelainsäure und ihren Derivaten, 1-Dimethyl-4-azasteroid, insbesondere 17-$\beta$-N,N-Diethylcarbamoyl-4-methyl-4-aza-5-$\alpha$-androstan-3-on, oder auch einen Extrakt von Serenoa repens.

16. Zusammensetzung nach einem der Ansprüche 13 bis 15, dadurch gekennzeichnet, daß die Braunelle Prunella Vulgaris L. oder Gemeine Braunelle ist.

17. Zusammensetzung nach einem der Ansprüche 13 bis 16, dadurch gekennzeichnet, daß der Extrakt von Braunelle oder dem Saponin von Braunelle durch Extraktion aus der ganzen Pflanze oder vorzugsweise aus den an der Luft befindlichen Teilen von Braunelle erhalten wird.

18. Zusammensetzung nach einem der Ansprüche 13 bis 17, dadurch gekennzeichnet, daß der Braunelle-Extrakt aus einer primären Extraktion von Trockenmaterial, das vorzugsweise aus an der Luft befindlichen Teilen von Braunelle besteht, mit einem Lösungsmittel, ausgewählt aus der Gruppe bestehend aus Wasser, Alkoholen mit vorzugsweise 1 bis 4 Kohlenstoffatomen, chlorierten Lösungsmitteln mit vorzugsweise 1 bis 2 Kohlenstoffatomen, organischen Estern mit vorzugsweise 3 bis 6 Kohlenstoffatomen, oder mit einem gemischten Lösungsmittel auf der Basis einer beliebigen Mischung der angegebenen Lösungsmittel erhalten wird.

19. Zusammensetzung nach Anspruch 18, dadurch gekennzeichnet, daß das Lösungsmittel der primären Extraktion ausgewählt wird aus der Gruppe bestehend aus Wasser, Methanol, Ethanol, einer Methanol-Wasser-Mischung oder einer Ethanol-Wasser-Mischung, Chloroform, Dichlormethan, wobei dieses Lösungsmittel vorzugsweise aus Wasser oder Methanol ausgewählt wird.

20. Verfahren zur kosmetischen Behandlung zur Bekämpfung der Effekte der Alterung auf die Epidermis oder Hautanhangsgebilde, zur Bekämpfung von Entzündungen, insbesondere durch Ultraviolett-Strahlen hervorgerufenem Erythem, zur Verbesserung oder Aufrechterhaltung eines guten funktionellen Zustands der Haut oder Hautanhangsgebilde, zur Prävention oder Behandlung trockener Haut, oder zur Behandlung von durch die Wirkung freier Radikale verursachten Störungen, dadurch gekennzeichnet, daß es das topische Aufbringen zumindest eines Extrakts von Braunelle oder zumindest einem Saponin von Braunelle in einer zum Erzielen des gewünschten Effekts wirksamen Menge, gegebenenfalls eingeschlossen in einem kosmetisch annehmbaren Exzipienten, umfaßt.

21. Verfahren nach Anspruch 20, dadurch gekennzeichnet, daß der Extrakt von Braunelle oder zumindest einem Saponin von Braunelle wie in einem der Ansprüche 4 bis 12 definiert ist.